(19) **Europäisches Patentamt / European Patent Office / Office européen des brevets**

(11) **EP 1 753 494 B1**

(12) **EUROPEAN PATENT SPECIFICATION**

(45) Date of publication and mention of the grant of the patent:
**23.08.2017 Bulletin 2017/34**

(21) Application number: **05736879.7**

(22) Date of filing: **22.04.2005**

(51) Int Cl.:
**A61M 16/01** *(2006.01)*

(86) International application number:
**PCT/EP2005/004353**

(87) International publication number:
**WO 2005/102432 (03.11.2005 Gazette 2005/44)**

(54) **APPARATUS FOR CHANGING THE CONCENTRATION OF A TARGET GAS AT THE BLOOD COMPARTMENT OF A PATIENT'S LUNG DURING ARTIFICIAL VENTILATION**

GERÄT ZUR VERÄNDERUNG DER KONZENTRATION EINES ZIELGASES IM BLUTKOMPARTIMENT DER LUNGE EINES PATIENTEN BEI DER KÜNSTLICHEN BEATMUNG

APPAREIL PERMETTANT DE MODIFIER LA CONCENTRATION EN GAZ CIBLE AU NIVEAU DU COMPARTIMENT DU SANG D'UN POUMON D'UN PATIENT PENDANT UNE VENTILATION ARTIFICIELLE

(84) Designated Contracting States:
**AT BE BG CH CY CZ DE DK EE ES FI FR GB GR HU IE IS IT LI LT LU MC NL PL PT RO SE SI SK TR**

(30) Priority: **23.04.2004 EP 04009688**
**22.10.2004 EP 04025174**
**10.02.2005 EP 05002840**

(43) Date of publication of application:
**21.02.2007 Bulletin 2007/08**

(73) Proprietors:
• **Böhm, Stephan, Dr.**
**21481 Lauenburg (DE)**
• **Tusman, Gerardo, Dr.**
**7600 Mar del Plata, Buenos Aires (AR)**
• **Manegold, Christoph**
**40549 Düsseldorf (DE)**

(72) Inventors:
• **Böhm, Stephan, Dr.**
**21481 Lauenburg (DE)**
• **Tusman, Gerardo, Dr.**
**7600 Mar del Plata, Buenos Aires (AR)**

• **Manegold, Christoph**
**40549 Düsseldorf (DE)**

(74) Representative: **Hasler, Erich et al**
**c/o Riederer Hasler & Partner**
**Patentanwälte AG**
**Elestastrasse 8**
**7310 Bad Ragaz (CH)**

(56) References cited:
**EP-A- 0 653 183     EP-A- 0 745 405**
**WO-A-00/44427     WO-A-86/05992**
**WO-A-98/41266     US-A- 4 596 246**

• **TUSMAN GERARDO ET AL: "Alveolar recruitment improves ventilatory efficiency of the lungs during anesthesia." August 2004 (2004-08), CANADIAN JOURNAL OF ANAESTHESIA = JOURNAL CANADIEN D'ANESTHESIE. 2004 AUG-SEP, VOL. 51, NR. 7, PAGE(S) 723 - 727 , XP009050798 ISSN: 0832-610X cited in the application the whole document**

## Description

The invention refers to an apparatus for changing the concentration of a target gas at the blood compartment of a patient's lung from an actual target gas concentration to a desired target gas concentration during artificial ventilation with an inspiratory gas composition by a respirator being controlled via a set of ventilation parameters.

The main function of the lung is gas exchange between atmospheric and blood gases where oxygen is absorbed into the blood and carbon dioxide, a product of body metabolism, is eliminated.

For maintaining this functioning, a lung needs to keep its normal morphology. Any 3D-morphological change will be related to an abnormal gas ventilation and blood perfusion distribution inside it. As a consequence, the alveolar-capillary membrane i.e. the lung zone where gas exchange takes place, cannot work optimally. In other words, any distortion of the normal ventilation and perfusion relationship affects normal gas exchange and a single patient will suffer from hypoxemia (decrease in arterial oxygenation).

Therefore, a perfect ventilation and perfusion relationship (V/Q) inside the alveoli is needed for a normal lung function. Any variation from the ideal value of 1 causes a deterioration of the gas exchange due a mismatching between these two functions.

During anaesthesia the patient's lung is filled with an inspiratory gas composition consisting of a fresh gas and possibly a fraction of re-breathed gas. The fraction of re-breathed gas is added only in semi-closed or closed circle breathing systems, whereas in open breathing systems the inspiratory gas composition consists purely of fresh gas. The fresh gas is composed of the target gas, i.e. the anaesthetic agent, oxygen and a carrier gas, i.e. nitrous oxide, helium or air. Inhalatory anaesthetic agents like halothane, isofluorane and sevofluorane are widely used in anaesthesia. These vapors enter the human beings by means of ventilation, delivered by an anaesthesia machine. The inhalatory agents reach the blood by diffusion through the alveolar-capillary membrane and are transported by the blood to the central nervous system. Diffusion is a passive transport through a membrane due to a partial pressure gradient. This means that inhalatory anaesthetic molecules go from the side with higher partial pressure to the side of the membrane with lower pressure. Firstly, during anaesthesia induction, where tissue anaesthetic concentration is zero, anaesthetic molecules go from the alveolar compartment (high concentration) to the blood (low concentration). In the opposite way, at the end of surgery when anaesthetic agent is withdrawn, anaesthetic concentration is higher at the blood compartment so that molecules follow an inverse way and are eliminated by breathing.

For example patent application EP 0 745 405 A1 discloses an anaesthetic system, which allows switching between an open mode and a re-breathing mode. When the breathing circuit is connected as a re-breathing system and a change in anaesthetic concentration or in type of anaesthetic is initiated, the switching unit automatically sets the breathing circuit as an open system for a predetermined period of time, the re-breathing mode being reset thereafter.

However, general anaesthesia and mechanical ventilation have a negative effect on the respiratory system. Thus both, respiratory mechanic and gas exchange through the alveolar-capillary membrane, deteriorate within 5 minutes from anaesthesia induction. This pathologic phenomenon is caused by a loss of gas volume inside the lungs due to closing of normally aerated lung regions, known as "lung collapse".

Recently, ventilatory recruitment maneuvers have been developed to solve the "lung collapse" problem in healthy and sick lungs. Recruitment maneuvers consist of a controlled increment in airway pressure until a point where the airway opening pressure is reached (the opening airway pressure of the lung is the airway pressure at which the closed units of the lung start opening). Afterwards, mechanical ventilation reassumes baseline ventilation with a level of positive end-expiratory pressure (PEEP) higher than the lung's closing pressure (i.e. airway pressure where opened units start closing, again).

In Tusman et.al.: "Alveolar Recruitment Strategy improves arterial oxygenation during general anaesthesia", British Journal of Anaesthesia 82(1) 8-13(1999), and in Tusman et. al.: "Alveolar recruitment strategy increases arterial oxygenation during one-lung ventilation", Annals of Thoracic Surgery 73:1204-1209 (2002) and in Tusman et.al.: "Effects of recruitment maneuver on atelectasis in anesthetized children", Anesthesiology 98:14-22 (2003) and in Tusman et.al.: "Lung recruitment improves the efficiency of ventilation and gas exchange during one-lung ventilation anesthesia", Anesthesia Analgesia 98: 1604-1609 (2004) and in Tusman et.al.: "Deadspace analysis before and after lung recruitment", Canadian Journal of Anesthesia 51:718-722 (2004) a recruitment maneuver is described which is used systematically for anesthetized patients. This maneuver has been useful to normalize lung volumes and gas exchange. Taking into account the above explanations, the alveolar recruitment strategy normalizes gas exchange because it improves ventilation and perfusion distribution within lungs, restoring an adequate V/Q relationship.

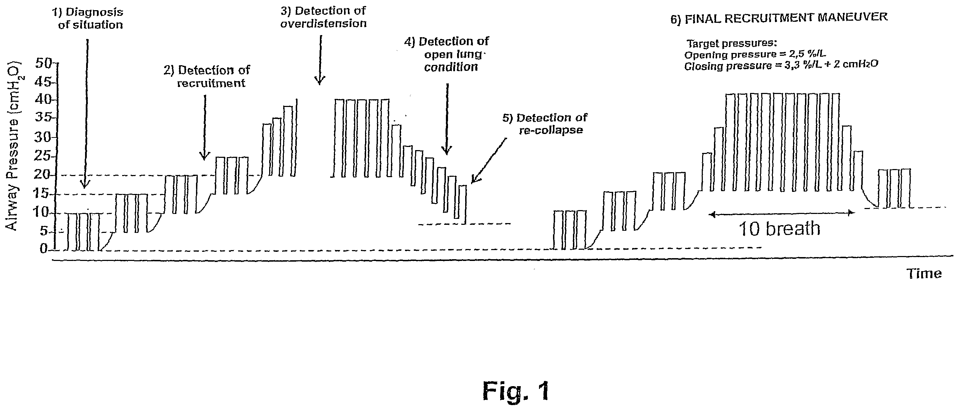

By way of an example, Fig. 1 shows a typical recruitment maneuver in detail. As shown in Fig. 1, the recruitment maneuver is carried out on the basis of a pressure controlled ventilation and uses two pressure levels, namely the peak inspiratory pressure (PIP) during inspiration and the positive end-expiratory pressure (PEEP) during expiration. Before the final recruitment maneuver takes place, the alveolar opening pressure and the alveolar closing pressure have to be identified. In a first step (step 1), PIP and PEEP are stepwise increased by means of an incremental limb until the alveolar opening pressures have been detected with regard to PIP and PEEP (steps 2 and 3). The alveolar opening pressure with regard to PIP is usually about 40 cmH$_2$O in normal lungs and in the range of 55-60 cmH$_2$O in sick lungs. After a successful alveolar opening, a decremental limb or stepwise decrease of PIP and PEEP is done (step 4) to determine the alveolar closing pressure (step 5). After having identified the pressures for alveolar opening and alveolar closing, the final recruitment maneuver (step 6) is done with these new target pressures over 10 breaths and PEEP is set above the alveolar closing pressure to avoid pulmonary re-collapse. For example, PEEP is set 2 cmH$_2$O above the alveolar closing pressure, i.e.

$$PEEP = PEEP_{close} + 2 \ cmH_2O$$

This alveolar recruitment strategy is used to ventilate patients with normal lungs as well as those with an acute lung disease in order to keep the lung open in case of a lung collapse. In other words, the alveolar recruitment strategy is applied for improving the gas exchange characteristic of a lung and thus to improve the mechanical behaviour of the patient's lung during artificial ventilation.

However, despite these efforts there remain various negative effects on the patient's body and in particular on the patient's respiratory system due to general anaesthesia.

Therefore, it is an object of the invention to decrease the negative effects of general anaesthesia during artificial ventilation even further.

The above mentioned object is solved by an apparatus according to claim 1, i.e. an apparatus for changing a concentration of a target gas at a blood compartment of a patient's lung from an actual target gas concentration to a desired target gas concentration during artificial ventilation with an inspiratory gas composition, the apparatus including a respirator for ventilating the inspiratory gas composition to a patient's lung, comprising

target gas varying means for varying the fraction of the target gas (S2) supplied to the inspiratory gas composition,

re-breathed gas varying means for varying the fraction of the re-breathed gas (S3) supplied to the inspiratory gas composition,

parameter varying means for varying a set of ventilation parameters (S1) being responsible for a ventilated lung volume, and

controlling means for controlling the target gas varying means, the re-breathed gas varying means and the parameter varying means, characterized in that

a) the target gas is an anaesthetic agent,

b) the lung is ventilated in a first ventilation stage by setting a fraction of target gas and a fraction of re-breathed gas, wherein said setting results in the actual target gas concentration, and wherein said set of ventilation parameters comprises at least a first peak inspiratory pressure and a first positive end-expiratory pressure,

c) the lung is ventilated in a second ventilation stage,

- wherein the set of ventilation parameters is based on a time-varying second peak inspiratory pressure above the first peak inspiratory pressure and a time-varying second positive end-expiratory pressure above the first positive end-expiratory pressure for yielding an increased ventilated lung volume compared to the first ventilation stage, and

- wherein on the basis of the increased ventilated lung volume the fraction of target gas, or the fraction of re-breathed gas, or a combination thereof, is varied such that the target gas concentration is changed towards the desired target gas concentration.

Further disclosed is a method for changing the concentration of a target gas at the blood compartment of a patient's lung from an actual target gas concentration to a desired target gas concentration during artificial ventilation with an inspiratory gas composition by a respirator being controlled via a set of ventilation parameters, by varying the fraction of the target gas supplied to the inspiratory gas composition, and/or the fraction of the re-breathed gas supplied to the inspiratory gas composition, and/or the set of ventilation parameters being responsible for the ventilated lung volume, wherein

a) the lung is ventilated in a first ventilation stage by setting a fraction of target gas, a fraction of re-breathed gas and a set of ventilation parameters, wherein said setting results in the actual target gas concentration, and

b) the lung is ventilated in a second ventilation stage in which at least once

- the set of ventilation parameters is varied for yielding an increased ventilated lung volume compared to the first ventilation stage and

- on the basis of the increased ventilated lung volume the fraction of target gas and/or the fraction of re-breathed gas is varied such that the target gas concentration is changed towards the desired target gas concentration.

The invention makes use of the fact that gas exchange during ventilation can be improved for all inhaled gases including anaesthetic agents when the ventilated lung volume is temporarily increased. The invention has recognized that the exchange of anaesthetic agents at the alveolar-capillary membrane can be improved on the basis of an increased ventilated lung volume, e.g. during and after an alveolar recruitment strategy due to normalization in V/Q relationship. This fact has an important clinical and economical meaning. For the clinical world, an improvement in gas exchange efficiency allows a faster anaesthesia induction, adjustment and emergence. For the economical world, an improved efficiency of gas exchange means that a lower amount of anaesthetic agents is needed for a single anaesthesia, thus decreasing hospital costs.

According to the invention, it has to be distinguished between a first ventilation stage and a second ventilation stage for changing the concentration of a target gas at the blood compartment from an actual target gas concentration to a desired gas concentration. The steady state of the first ventilation stage corresponds to the actual target gas concentration of the blood compartment. The aim is now to change the concentration of the target gas at the blood compartment towards the desired target gas concentration during the second ventilation stage. According to the invention, during the second ventilation stage the alveolar recruitment strategy is applied wherein at the same time the inspiratory gas composition is controlled such that the second ventilation stage yields a change of the actual target gas concentration of the blood compartment towards the desired target gas concentration of the blood compartment. However, one technical difficulty of alveolar recruitment strategy regarding inhalatory anaesthetic delivery to the patients is a dilution effect. The alveolar recruitment strategy demands a high-flow to fill the gain of lung volume (recruited volume) while the target airway pressures are reached. Application of this additional volume is hindered due to the restricted capacity of the tidal volume generating modules (bag-in-bellow, bag-in-bottle, piston driven ventilator) of traditional anaesthesia machines. Additionally, dilution effects can be caused by the re-breathed gas in a semi-closed or closed circle system or by extensive use of the oxygen flush function. Thus, an amount of a volume of gas without anaesthetic agents enters into the lung and into the anaesthetic circuit, diluting the anaesthetic gas concentration at the alveolar-capillary membrane. Obviously, this dilution effect wastes anaesthetic agents and increases the chance of an inadvertent recovery or awareness of the patient.

Therefore, the invention controls the inspiratory gas composition during the second ventilation stage such that the change from the actual target gas concentration towards the desired target gas concentration is supported. In particular, when a sudden increase of the ventilated lung volume occurs due to the increase of the peak inspiratory pressure and the positive end-expiratory pressure, the increased volume is filled with a gas which yields a change of the actual target gas concentration of the blood compartment towards the desired target gas concentration of the blood compartment. More specifically, it has to be ensured that the additional gas which is filled in the increased lung volume is of the type of the desired target gas concentration which has to be achieved in the blood compartment.

In practice, the invention can be realized by switching between the usual closed ventilation system and an adapted open ventilation system. In the first ventilation stage, a closed ventilation system can be applied. This means, that re-breathed gases are re-circulated in the system which makes the system cost-efficient because anaesthetic agents can be re-used. However, it also has to be observed that a re-breathing might cause a dilution effect so that the fraction of the specific target gas supplied to the inspiratory gas composition might vary within a certain range.

On the other hand, in the second ventilation stage an open ventilation system is more appropriate for a well controlled variation of the fraction of target gas. It has to be observed that due to the increased lung volume a closed ventilation system in the second ventilation stage causes a considerable dilution effect when supplying the additional gas (usually air) to the increased lung volume, However, having an open ventilation system it is possible to fill the increased lung volume with the appropriate gas, e. g. the desired target gas itself. At the same time, the expired gases coming from the patient can be discarded in order not to dilute the inspired gases. This means, that with an open ventilation system the fraction of target gas supplied during the second ventilation stage can be controlled precisely. However, a disadvantage is the fact that the open ventilation system cannot be operated as cost-efficient as the closed ventilation system.

It should be noted, that in fact the steps comprising the second ventilation stage can be applied multiple times consecutively in order to make use of an overshoot. Usually, an overshoot within the second ventilation stage is not desired because the actual target gas concentration at the blood compartment might deviate too much from the desired target gas concentration which might put the patient's life at risk. However, the beginning of an overshoot might be induced during the second ventilation stage, whereas subsequently this overshoot is cushioned by counter-acting against the overshoot. Such a technique can be used to accelerate the change from the actual target gas concentration towards the desired gas concentration even further. From the field of control engineering this kind of overshoot technique is well-known, for example from the so-called PID-controller.

For some cases, the ventilation will finish after having reached the desired target gas concentration during the second ventilation stage or repetitions of the steps comprising the second ventilation stage which are applied one after the other. However, in most of the cases a third ventilation stage will be required in which a steady state of the desired target gas concentration is reached. Therefore, according to a preferred aspect of the invention, the lung is ventilated in a third ventilation stage by setting a fraction of target gas, a fraction of re-breathed gas and a set of ventilation parameters, wherein the set of ventilation parameters yields a decreased ventilated lung volume compared to the second ventilation stage and wherein said setting results in the desired target gas concentration.

According to the explanations above, a closed ventilation system is again appropriate to be applied during the third ventilation stage.

In practice, the controlling means according to the invention for controlling the parameter varying means, the target gas varying means and the re-breathed gas varying means can comprise a switch for switching between a closed ventilation system (first ventilation stage) and an open ventilation system (second ventilation stage) and again a closed ventilation system (third ventilation stage).

According to the invention the target gas is an anaesthetic agent. This means, that the invention applies to the field of anaesthesia where the concentration of the anaesthetic agent at the blood compartment has to be changed and where it is advantageous to reduce the time for performing such a change.

This means, that the invention can be applied both to a wash-in process of anaesthesia and to a wash-out process of anaesthesia. If the invention is applied to a wash-in process of anaesthesia the target gas supplied in the first ventilation stage is an anaesthetic agent corresponding to a state of shallow or no general anaesthesia and the target gas supplied in the second ventilation stage is an anaesthetic agent corresponding to a state of deeper general anaesthesia. On the other hand, if the invention is applied to a wash-out process of anaesthesia the target gas supplied in the first ventilation stage is an anaesthetic agent corresponding to a state of deeper general anaesthesia and the target gas supplied in the second ventilation stage is an anaesthetic agent corresponding to a state of shallow or no general anaesthesia.

The alveolar recruitment strategy is a well-tested method for temporarily increasing the ventilated lung volume. When applying the alveolar recruitment strategy the set of ventilation parameters during the second ventilation stage has to be adjusted accordingly. In general, the set of ventilation parameters of the first ventilation stage is based on a first peak inspiratory pressure and a first positive end-expiratory pressure. Furthermore, the set of ventilation parameters of the second ventilation stage is based on a time-varying second peak inspiratory pressure above the first peak inspiratory pressure and a time-varying second positive end-expiratory pressure above the first positive end-expiratory pressure. If a third ventilation stage as mentioned above is applied, the set of ventilation parameters of the third ventilation stage is based on a third peak inspiratory pressure, which is lower than the maximum of the time-varying second peak inspiratory pressure and a third positive end-expiratory pressure, which is lower or equal to the maximum of the time-varying second positive end-expiratory pressure.

With reference to Fig. 1, the set of ventilation parameters characterizing the first ventilation stage is applied in the beginning of the final recruitment maneuver (1 breath cycle comprising 3 breaths), the set of ventilation parameters characterizing the second ventilation stage is applied in the middle of the final recruitment maneuver (1 breath cycle comprising 10 breaths and 2 breath cycles in advance comprising each 3 breaths), and the set of ventilation parameters characterizing the third ventilation stage is applied in the end of the final recruitment maneuver (1 breath cycle comprising 3 breaths). It should be noted, that Fig. 1 and the corresponding description relate to one isolated example of an ARS only. Different ways of performing an ARS, in particular with respect to the number of breath cycles and breaths per cycle, can be employed within the method disclosed in the present specification. Another mode of ventilation for achieving an increased volume is a volume controlled ventilation. This mode has the advantage that the ventilated volume remains constant and that all changes of the lung status can be related to changes within the alveoli. In general, any possible mode of ventilation as well as any combination thereof can be applied according to the invention.

Other objects and features of the invention will become apparent by reference to the following specification, in which

Fig. 1    shows a sample plot of the airway pressures over time of a typical recruitment maneuver,

Fig. 2    shows a plot of the time constant (TAU) concept,

Fig. 3    shows a schematic representation of the underlying concept of the invention,

Fig. 4    A,B,C show schematic representations of an anaesthesia system in a re-breathing and non-re-breathing mode according to the prior art,

Fig. 5    shows a plot of the expired anaesthetic fraction during start of anaesthesia,

Fig. 6    shows a plot of the expired anaesthetic fraction during end of anaesthesia,

Fig. 7    shows a plot of the gas kinetic during alveolar recruitment strategy (ARS),

Fig. 8    shows a schematic representation of the lung volumes for different gas volumes,

Fig. 9    shows a table of the combinations according to the invention between the set of ventilation parameters, the fraction of target gas and the fraction of re-breathed gas on the one hand and the different ventilation stages on the other hand,

Fig. 10  illustrates the operation of the alveolar recruitment strategy according to the prior art,

Fig. 11  illustrates the operation of the invention during a wash-in process, and

Fig. 12  illustrates the operation of the invention during a wash-out process.

Fig. 1 has been explained in the introductory part.

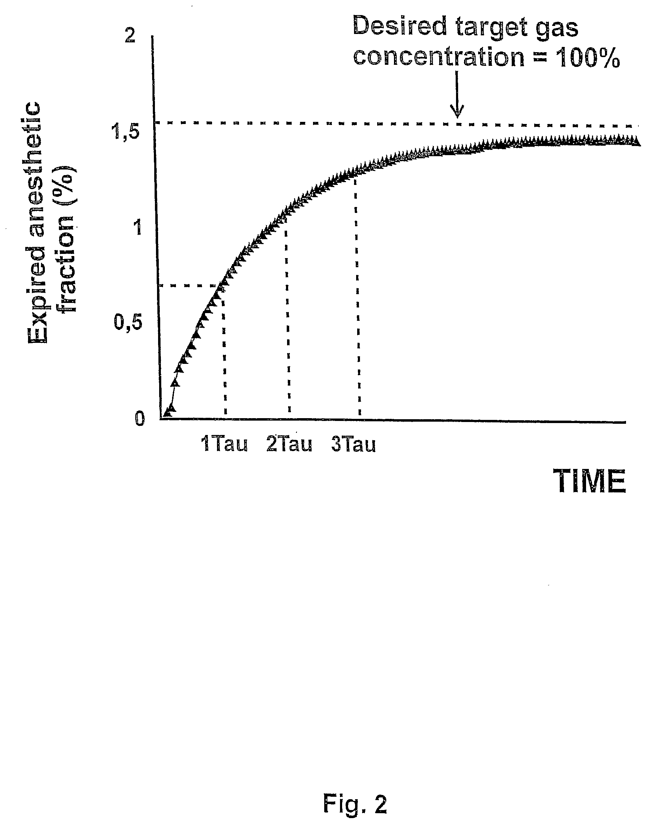

Fig. 2 shows a plot of the time constant (TAU) concept: The graphic shows the expired fraction of isofluorane being the target gas in this example against time using a semi-closed system. The first horizontal broken line indicates a concentration of 50% of the desired anaesthetic gas concentration. The corresponding first vertical broken line indicates the time required to reach this 50% concentration. This time period is called the time constant or TAU. After a time of 3 x TAU more than 90% of the desired concentration is reached. The expired anaesthetic fraction represents the fraction of anaesthetic agent present in the gas being discarded from the patient. While this can be easily measured at the airway opening on-line and non-invasively, corresponding measurements of the target gas concentration of the blood compartment are considerably more difficult to perform. However, recordings of the expired anaesthetic fraction can be seen as a qualitative indication of the target gas concentration of the blood compartment, at least with respect to its variation.

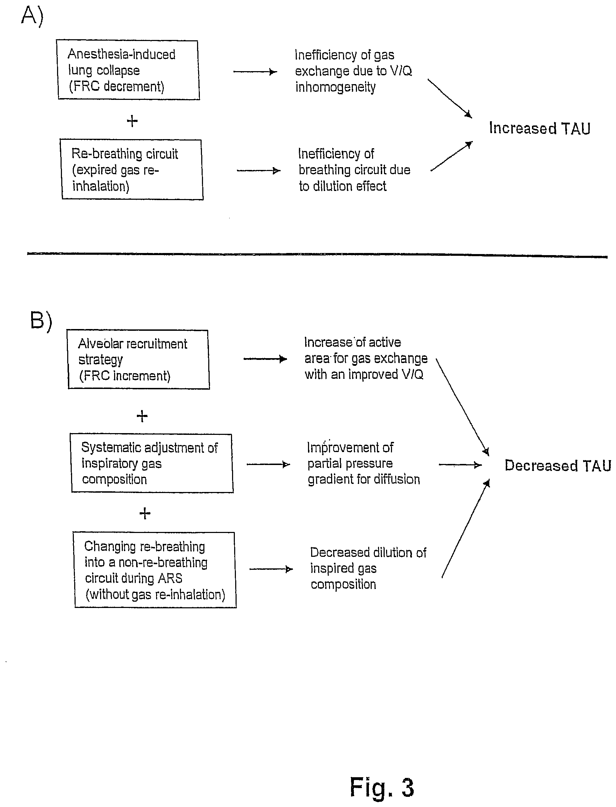

Fig. 3 shows a schematic representation of the underlying concept of the invention.

A) shows the concept of a standard anaesthesia machine. Both anaesthesia-induced lung collapse and re-breathing anaesthetic circuit increase TAU according to Fig. 2.

B) shows a new device according to the invention with a novel method and system to lower TAU. Due to the combination of alveolar recruitment strategy, a systematic adjustment of inspiratory gas composition and a changing of a closed ventilation system to an open ventilation system, i.e. changing from re-breathing to non-re-breathing during or before/after the alveolar recruitment strategy.

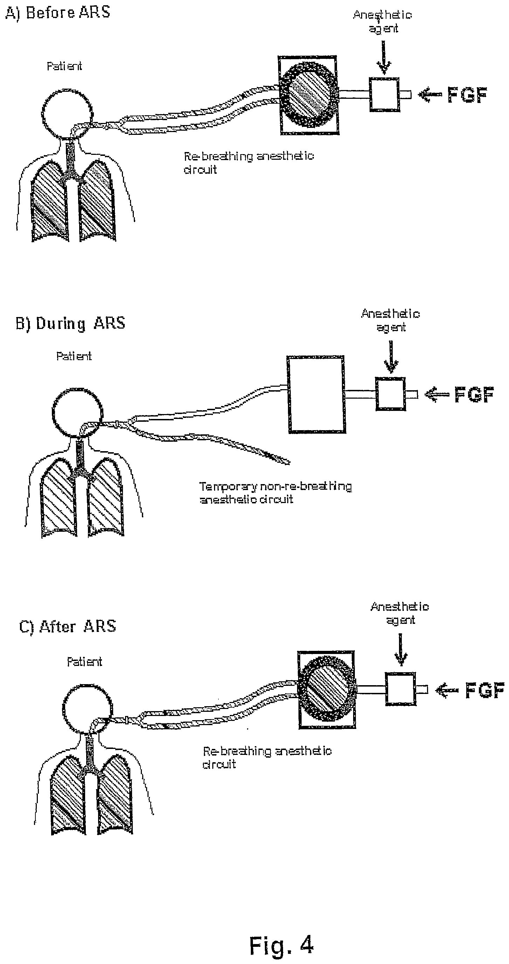

Fig. 4 shows a schematic representation of a typical anaesthesia system and its sequential modifications (A,B,C) according to the invention. While an alveolar recruitment maneuver is performed, the anaesthetic system is transformed from a re-breathing (A) into a non-re-breathing (so called "open") system (B) where re-breathing of expired gas is eliminated. Afterwards, the anaesthetic circuit is transformed back into a re-breathing (so called "closed or semi-closed") system (C).

A) shows a schematic representation of a re-breathing anaesthesia system. A fresh gas flow (FGF) is delivered into the patient through the inspired limb of the anaesthesia circuit. Expired gases return to the system through the expired limb of the anaesthesia circuit (striped area), diluting the fresh gas during the next inspiration (partially striped areas). This "dilution" effect increases the time constant (TAU) for any change in the concentration of the target gas within the inspired gas composition of anaesthetics.
B) shows a schematic representation of a non-re-breathing anaesthesia system. A fresh gas flow (FGF) is delivered into the patient through the inspired limb of the anaesthesia circuit while expired gases are discarded. During the next inspiration pure fresh gas is delivered to the patient. There is no "dilution" effect. Thus, the time constant (TAU) for any change in the concentration of the target gas within the inspired gas composition of anaesthetics is lower than in A.
C) shows the same schematic representation of a re-breathing anaesthesia system as under A. A fresh gas flow (FGF) is delivered into the patient through the inspired limb of the anaesthesia circuit. Expired gases return to the system through the expired limb of the anaesthesia circuit (striped area), diluting the fresh gas during the next inspiration (partially striped areas). This "dilution" effect increases the time constant (TAU) for any change in the concentration of the target gas within the inspired gas composition of anaesthetics.

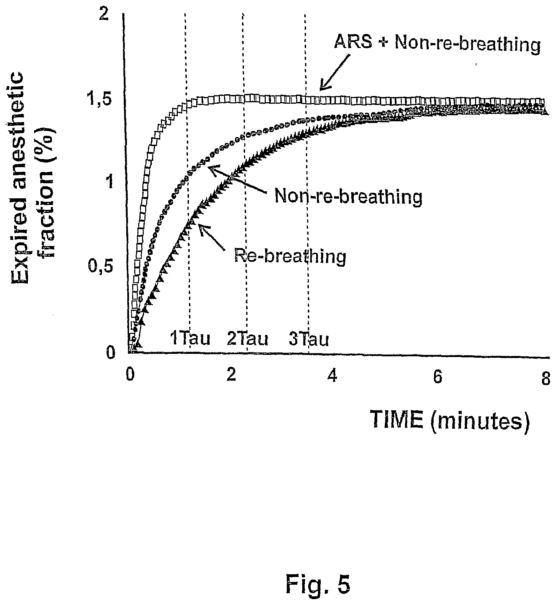

Fig. 5 shows a plot of the concentration of an anaesthetic agent in the expiratory gas composition, namely isofluorane which is the target gas in this example, during start of anaesthesia with wash-in of anaesthetic agent (desired concentration of the target gas in the expiratory gas composition = 1.5%). The graphic shows the concentration of isofluorane in the expiratory gas composition against time using a common re-breathing "semi-closed" system (black triangles), an "open system" without re-breathing (black dots) and an alveolar recruitment maneuver (ARS) in conjunction with a non-re-breathing system (open squares). TAU is longer in the re-breathing circuit than in the two non-re-breathing systems. However, ARS in combination with a non-re-breathing decreases TAU even more, thus reaching the desired concentration of the target gas in the expiratory gas composition faster. Although, the concentration of the target gas in the expiratory gas composition was measured in the airway opening, a qualitatively similar result can be expected for the target gas concentration of the blood compartment.

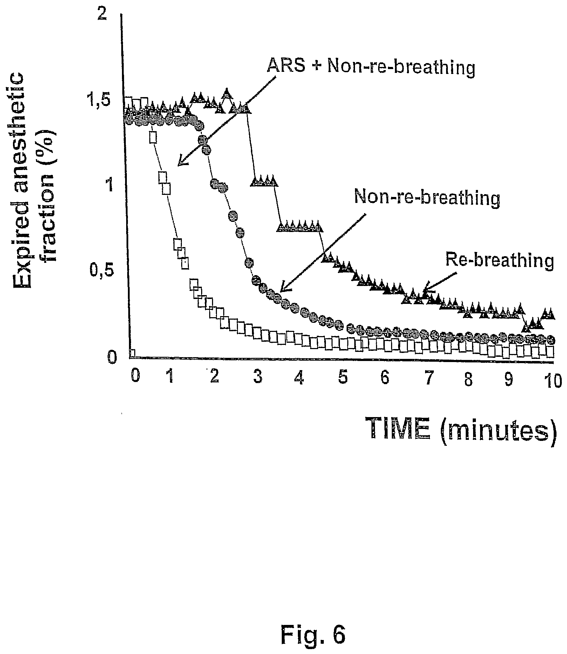

Fig. 6 shows a plot of the concentration of an anaesthetic agent in the expiratory gas composition, namely isofluorane which is the target gas in this example, during end of anaesthesia with a wash-out of anaesthetic agent (concentration of the target gas in the inspiratory gas composition = zero, desired concentration of the target gas in the expiratory gas composition = zero). The graphic shows expired isofluorane fraction against time using a common re-breathing "semi-closed" system (filled triangles), an "open" system without re-breathing (filled dots) and an alveolar recruitment maneuver (ARS) in conjunction with a non-re-breathing system (open squares). TAU is longer in the re-breathing circuit compared to the non-re-breathing systems. ARS applied in a non-re-breathing system decreases TAU even more, thus reaching the desired target gas concentration faster. Again, the concentration of the target gas in the expiratory gas composition gives a qualitative indication of the target gas concentration of the blood compartment, in particular, if an expired target gas fraction of 0% is present, the target gas concentration of the blood compartment is as well 0%.

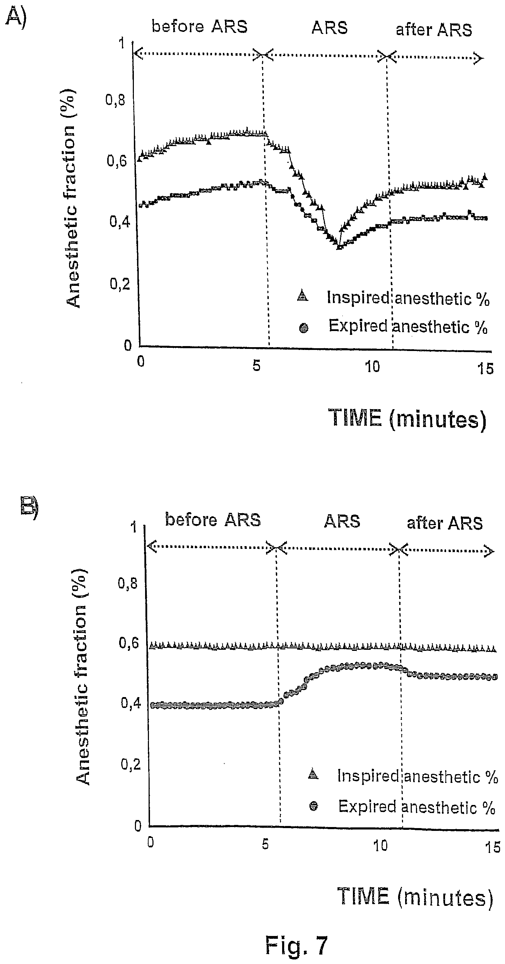

Fig. 7 shows a plot of the gas kinetic during alveolar recruitment strategy (ARS):

A) ARS performed in a semi-closed circuit, where re-breathing allows a dilution effect of anaesthetic gases (target gas). At the end, both inspiratory and expiratory gas compositions show target gas concentrations that reach a steady state at lower concentrations than before the ARS maneuver. Noticeably, the anaesthetic fraction of the inspired gas composition is reduced during the lung recruitment maneuver as a result of the dilution effect when increasing the lung volume. As a consequence, the anaesthetic fraction of the expired gas composition, and hence the actual target gas concentration of the blood compartment, is reduced as well. This effect is a problem within anaesthesia, e.g. inadvertent recovery or awareness of the patient, and it is the object of the invention to overcome this problem.

B) ARS without re-breathing, where a constant inspired gas composition is kept having a constant target gas concentration which corresponds to the desired target gas concentration of the blood compartment during and after the recruitment process. It can be noted that due to a better gas exchange obtained with a lung recruitment maneuver the difference between the target gas concentration in the inspiratory and in the expiratory gas composition is lower after ARS compared to the state before. This means that the invention makes anaesthesia more efficient.

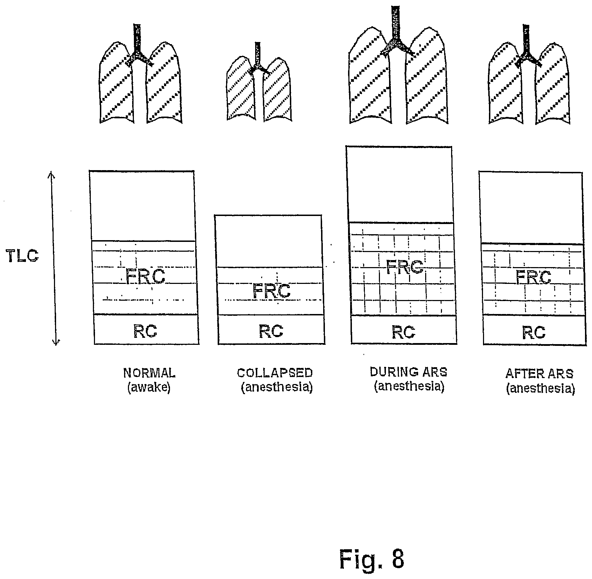

Fig. 8 shows a schematic representation of the lung volumes for different gas volumes in an awake patient, during anaesthesia as well as during and after the application of an alveolar recruitment strategy (ARS). Total lung capacity (TLC) is the volume of gas within lungs at end-inspiration. Functional residual capacity (FRC) is the volume of gas within lungs at end-expiration. It is reduced during anaesthesia due to lung collapse. The ARS restores normal lung volumes by recruiting previously collapsed lung units and is associated with normal gas exchange.

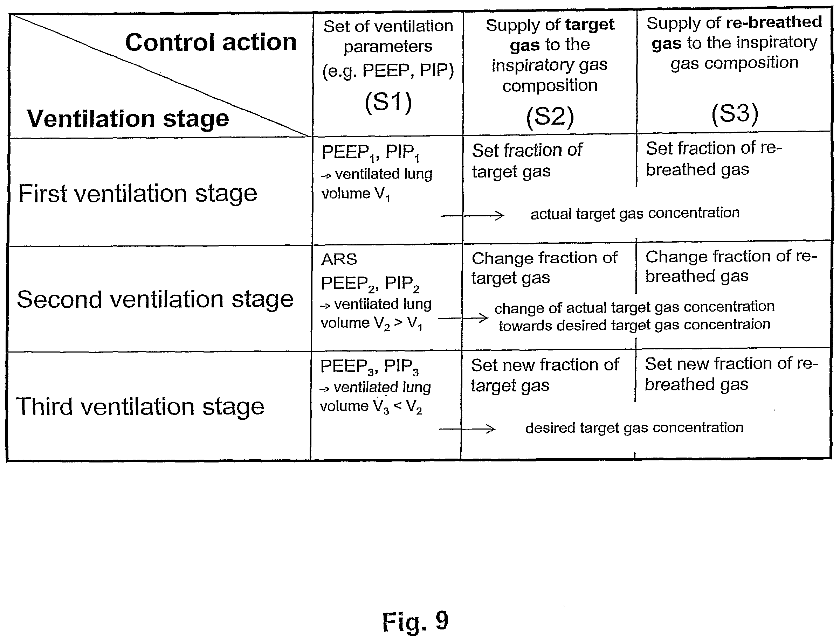

Fig. 9 shows a table of the combinations according to the invention between the set of ventilation parameters, the fraction of target gas and the fraction of re-breathed gas on the one hand and the different ventilation stages on the other hand. During the three ventilation stages the corresponding control actions or combinations thereof can be applied as already described above. The three control actions are based on the set of ventilation parameters (S1), the fraction of target gas supplied to the inspiratory gas composition (S2) and the fraction of re-breathed gas supplied to the inspiratory gas composition (S3). These three control actions can be used like control parameters known from the control theory to achieve the best performance of the change from the actual target gas concentration at the blood compartment to the desired target gas concentration at the blood compartment. This means that not necessarily all three actions have to be applied during one stage but that also only one or two control actions might be applied, where appropriate.

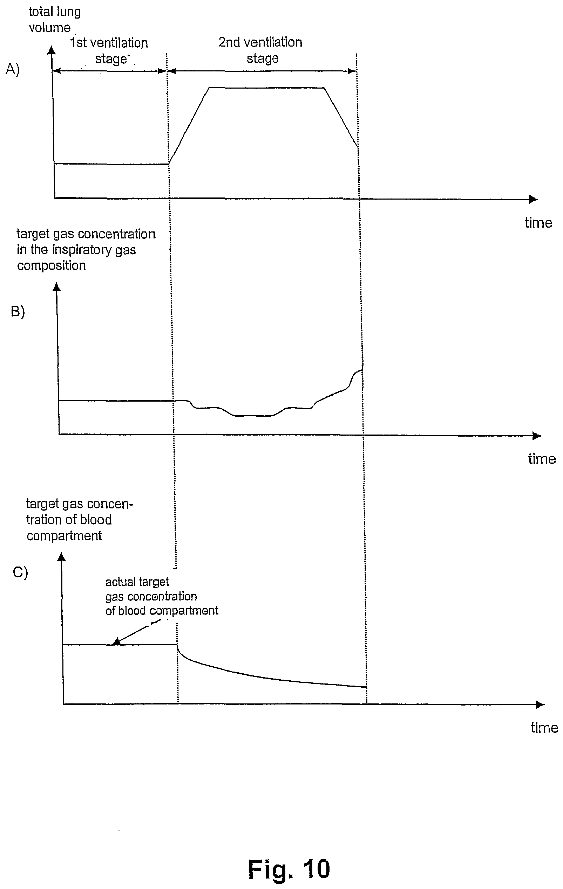

Fig. 10 illustrates the operation of the alveolar recruitment strategy according to the prior art. Shown are plots of the total lung volume, concentration of the target gas in the inspiratory gas composition and the target gas concentration of the blood compartment over the same time scale. In the first ventilation stage, before starting the lung recruitment maneuver, the total lung volume is small, while the target gas concentration of the inspiratory gas composition results in a certain target gas concentration of the blood compartment (steady state). Once the lung recruitment maneuver begins in the second ventilation stage, the lung volume increases. A conventional closed ventilation system is used so that a reduction of the target gas concentration of the blood compartment occurs during the second ventilation stage due to the dilution effect.

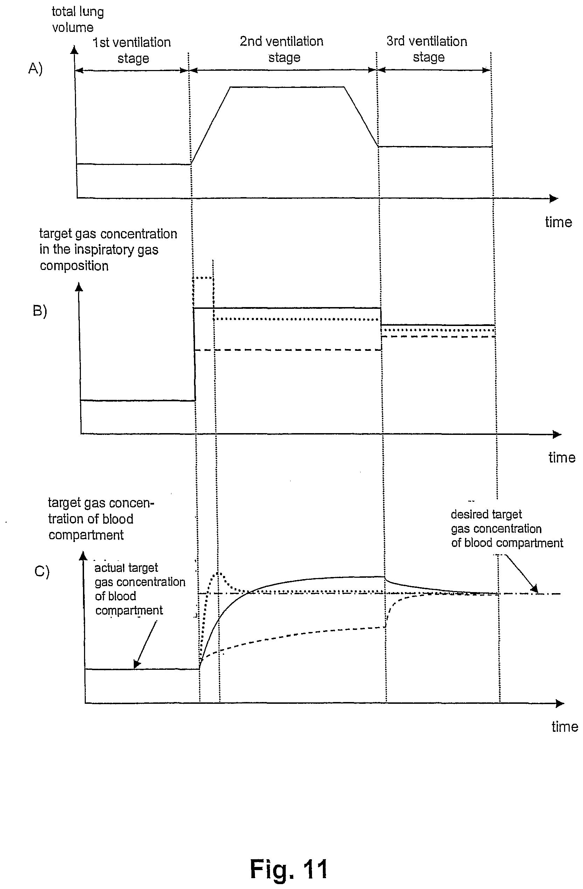

Fig. 11 illustrates the operation of the invention during a wash-in process. Shown are plots of the total lung volume, target gas concentration in the inspiratory gas composition and the target gas concentration of the blood compartment over the same time scale. In the first ventilation stage, before starting the lung recruitment maneuver, the total lung volume is small, while the target gas concentration of the inspiratory gas composition results in a certain target gas concentration of the blood compartment (steady state). Once the lung recruitment maneuver begins in the second ventilation stage, the total lung volume increases. According to the invention, the target gas concentration of the inspiratory gas composition within the second ventilation stage is modified by adjusting the fraction of target gas and the fraction of re-breathed gas supplied to the inspiratory gas composition in as such as to yield a change of the target gas concentration of the blood compartment towards the desired target gas concentration. As depicted in Fig. 11 c), this alteration of the target gas concentration of the blood compartment can be of various types, including an overshoot. Similarly, the variation of the concentration of the target gas in the inspiratory gas composition can be of various types and can include multiple variations within the second ventilation stage. In the third ventilation stage the concentration of the target gas in the blood compartment reaches the desired target gas concentration of the blood compartment in a steady state.

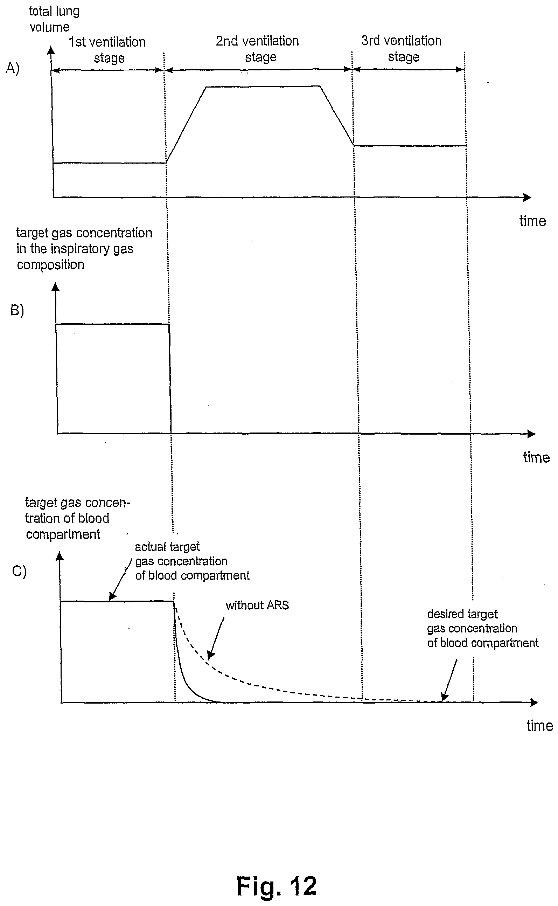

Fig. 12 illustrates the operation of the invention during a wash-out process. Shown are plots of the total lung volume, target gas concentration in the inspiratory gas composition and the target gas concentration of the blood compartment over the same time scale. The target gas shall be removed completely from the blood compartment. In the first ventilation stage, before starting the lung recruitment maneuver, the total lung volume is small, while the target gas concentration of the inspiratory gas composition results in a certain target gas concentration of the blood compartment (steady state). Once the lung recruitment maneuver begins in the second ventilation stage, the lung volume increases. According to the invention, the concentration of the target gas within the second ventilation stage is modified by adjusting the fraction of target gas and the fraction of re-breathed gas supplied to the inspiratory gas composition in as such as to yield a decrease of the target gas concentration of the blood compartment. Preferably, in a wash-out process the concentration of the target gas within the inspiratory gas composition is 0%. A wash-out process of the target gas without ARS would result in a slower withdrawal of the target gas from the blood compartment.

**Claims**

1. Apparatus for changing a concentration of a target gas at a blood compartment of a patient's lung from an actual target gas concentration to a desired target gas concentration during artificial ventilation with an inspiratory gas composition, the apparatus including a respirator for ventilating the inspiratory gas composition to a patient's lung, comprising
target gas varying means for varying the fraction of the target gas (S2) supplied to the inspiratory gas composition,
re-breathed gas varying means for varying the fraction of the re-breathed gas (S3) supplied to the inspiratory gas composition,
parameter varying means for varying a set of ventilation parameters (S1) being responsible for a ventilated lung volume, and
controlling means for controlling the target gas varying means,
the re-breathed gas varying means and the parameter varying means, **characterized in that**

    a) the target gas is an anaesthetic agent,
    b) the lung is ventilated in a first ventilation stage by setting a fraction of target gas and a fraction of re-breathed gas, wherein said setting results in the actual target gas concentration, and wherein said set of ventilation parameters comprises at least a first peak inspiratory pressure and a first positive end-expiratory pressure,
    c) the lung is ventilated in a second ventilation stage,

       - wherein the set of ventilation parameters is based on a time-varying second peak inspiratory pressure above the first peak inspiratory pressure and a time-varying second positive end-expiratory pressure above the first positive end-expiratory pressure for yielding an increased ventilated lung volume compared to the first ventilation stage, and
       - wherein on the basis of the increased ventilated lung volume the fraction of target gas, or the fraction of re-breathed gas, or a combination thereof, is varied such that the target gas concentration is changed towards the desired target gas concentration.

2. Apparatus according to claim 1, wherein the lung is ventilated in a third ventilation stage by setting a fraction of target gas, a fraction of re-breathed gas, a third peak inspiratory pressure, which is lower than the maximum of the time-varying second peak inspiratory pressure, and a third positive end-expiratory pressure, which is lower or equal to the maximum of the time-varying second positive end- expiratory pressure, for yielding a decreased ventilated lung volume compared to the second ventilation stage and wherein said setting results in the desired target gas concentration.

3. Apparatus according to one of claims 1 or 2, wherein during a wash-in process of anaesthesia the target gas supplied in the first ventilation stage is an anaesthetic agent corresponding to a state of shallow or no general anaesthesia and the target gas supplied in the second ventilation stage is an anaesthetic agent corresponding to a state of deeper general anaesthesia.

4. Apparatus according to any one of claims 1 to 3, wherein during a wash-out process of anaesthesia the target gas supplied in the first ventilation stage is an anaesthetic agent corresponding to a state of deeper general anaesthesia and the target gas supplied in the second ventilation stage is an anaesthetic agent corresponding to a state of shallow or no general anaesthesia.

**5.** Apparatus according to any one of claims 1 to 4, comprising further a switch for switching between a closed ventilation system and an open ventilation system in order to ventilate a lung in the first ventilation stage in a closed or semi-closed ventilation system, where a fraction of re-breathed gas is added in the breathing system, and in the second ventilation stage in an open ventilation system.

**6.** Apparatus according to claim 5, wherein the lung is ventilated in the third ventilation stage in a closed or semi-closed ventilation system.

**Patentansprüche**

**1.** Vorrichtung zur Änderung der Konzentration eines Zielgases auf der Blutseite der Lunge eines Patienten von einer aktuellen Zielgaskonzentration zu einer gewünschten Zielgaskonzentration während der künstlichen Beatmung mit einem Inspirationsgasgemisch, wobei die Vorrichtung einen Respirator zur Zufuhr des Inspiratiansgasgemisches zur Lunge eines Patienten aufweist, umfassend:

Zielgasänderungsmittel zur Änderung des Anteils an Zielgas (S2), das dem Inspirationsgasgemisch zugesetzt wird,
Expirationsgasänderungsmittel zur Änderung des Anteils an Expirationsgas (S3), das dem Inspirationsgasgemisch zugeleitet wird,
Parameteränderungsmittel zur Änderung einer Reihe von Beatmungsparametern (S1), die für ein belüftetes Lungenvolumen verantwortlich sind, und
Steuermittel zur Steuerung der Zielgasänderungsmittel, der Expirationsgasänderungsmittel und der Parameteränderungsmittel, **dadurch gekennzeichnet, dass**

a) das Zielgas ein Anästhetikum ist,
b) die Lunge in einer ersten Beatmungsstufe unter Einstellung eines Anteils an Zielgas und eines Anteils an Expirationsgas beatmet wird, wobei die genannte Einstellung zur aktuellen Zielgaskonzentration führt, und wobei die genannte Beatmungsparameterreihe mindestens einen ersten maximalen Beatmungsdruck enthält und einen ersten positiven endexpiratorischen Druck,
c) die Lunge in einer zweiten Beatmungsstufe beatmet wird,

- wobei die Reihe von Beatmungsparametern auf einem zeitlich veränderlichen, zweiten maximalen Beatmungsdruck, der höher als der erste maximale Beatmungsdruck ist, beruht, und einem zeitlich veränderlichen zweiten positiven endexpiratorischen Druck, der höher als der erste positive endexpiratorische Druck ist, um ein größeres belüftetes Lungenvolumen gegenüber der ersten Beatmungsstufe zu erreichen, und
- wobei auf der Basis des größeren belüfteten Lungenvolumens der Anteil an Zielgas oder der Anteil an Expirationsgas oder eine Kombination davon derart geändert ist, dass die Zielgaskonzentration zur gewünschten Zielgaskonzentration geändert wird.

**2.** Vorrichtung nach Patentanspruch 1, in der die Lunge in einer dritten Beatmungsstufe unter Einstellung eines Zielgasanteils, eines Expirationsgasanteils, eines dritten maximalen Beatmungsdruckes, der niedriger ist, als das Maximum des zeitlich veränderlichen zweiten maximalen Beatmungsdruckes, und eines dritten positiven endexpiratorischen Druckes, der niedriger als oder gleich dem Maximum des zeitlich veränderlichen zweiten positiven endexpiratorischen Druckes ist, beatmet wird, um ein kleineres belüftetes Lungenvolumen gegenüber der zweiten Beatmungsstufe zu erreichen, und wobei die genannte Einstellung zur gewünschten Zielgaskonzentration führt.

**3.** Vorrichtung nach einem der Patentansprüche 1 oder 2, in der in der Anflutungsphase der Anästhesie das in der ersten Beatmungsstufe zugesetzte Zielgas ein Anästhetikum ist, das einem Zustand flacher, nicht Allgemeinanästhesie entspricht, und das in der zweiten Beatmungsstufe verabreichte Zielgas ein Anästhetikum ist, das einem Zustand tieferer Allgemeinanästhesie entspricht.

**4.** Vorrichtung nach irgendeinem der Patentansprüche 1 bis 3, in der in der Abflutungsphase der Anästhesie das in der ersten Beatmungsstufe verabreichte Zielgas ein Anästhetikum ist, das einem Zustand tieferer Allgemeinanästhesie entspricht, und das in der zweiten Beatmungsstufe verabreichte Zielgas ein Anästhetikum ist, das einem Zustand flacher, nicht Allgemeinanästhesie entspricht.

**5.** Vorrichtung nach irgendeinem der Patentansprüche 1 bis 4, außerdem einen Schalter zum Umschalten zwischen einem geschlossenen Beatmungssystem und einem offenen Beatmungssystem umfassend, um eine Lunge in der ersten Beatmungsstufe in einem geschlossenen oder halbgeschlossenen Beatmungssystem zu beatmen, in dem dem Beatmungssystem ein Anteil an Expirationsgas zugeführt wird, und in der zweiten Beatmungsstufe in einem offenen Beatmungssystem.

**6.** Vorrichtung nach Patentanspruch 5, in der die Lunge in der dritten Beatmungsstufe in einem geschlossenen oder halbgeschlossenen Beatmungssystem beatmet wird.

## Revendications

**1.** Appareil pour changer une concentration d'un gaz cible dans un compartiment sanguin d'un poumon d'un patient à partir d'une concentration actuelle de gaz cible en une concentration souhaitée de gaz cible pendant une ventilation artificielle avec une composition de gaz inspiratoire, l'appareil comprenant un ventilateur pour ventiler la composition de gaz inspiratoire dans un poumon d'un patient comprenant :

des moyens pour faire varier le gaz cible pour faire varier la fraction de gaz cible (S2) fournie dans la composition de gaz inspiratoire,
des moyens pour faire varier le gaz inspiré à nouveau pour faire varier la fraction de gaz inspiré à nouveau fournie dans la composition de gaz inspiratoire,
des moyens pour faire varier les paramètres pour faire varier un ensemble de paramètres de ventilation (S1) qui sont responsables d'un volume du poumon ventilé et
des moyens de commande pour commander les moyens pour faire varier le gaz cible, les moyens pour faire varier le gaz inspiré à nouveau et les moyens pour faire varier les paramètres, **caractérisé en ce que**

a) le gaz cible est une substance anesthésique,
b) le poumon est ventilé dans une première étape de ventilation en réglant une fraction de gaz cible et une fraction de gaz inspiré à nouveau, ledit réglage résultant dans la concentration actuelle de gaz cible et ledit ensemble de paramètres de ventilation comprenant au moins une première pression inspiratoire de crête et une première pression expiratoire positive,
c) le poumon est ventilé dans une seconde étape de ventilation,

- cependant que l'ensemble de paramètres de ventilation se base sur une seconde pression inspiratoire de crête qui varie dans le temps au-dessus de la première pression inspiratoire positive et une seconde pression expiratoire positive qui varie dans le temps au-dessus de la première pression expiratoire positive pour produire un volume de poumon ventilé augmenté en comparaison avec la première étape de ventilation et
- cependant que sur la base du volume de poumon ventilé augmenté, la fraction de gaz cible ou la fraction de gaz inspiré à nouveau ou une combinaison de ceux-ci varie de telle manière que la concentration de gaz cible est changée vers la concentration souhaitée de gaz cible.

**2.** Appareil selon la revendication 1, le poumon étant ventilé dans une troisième étape de ventilation en réglant une fraction de gaz cible, une fraction de gaz inspiré à nouveau, une troisième pression inspiratoire de crête qui est inférieure au maximum de la seconde pression inspiratoire de crête qui varie dans le temps et une troisième pression expiratoire positive qui est inférieure ou égale au maximum de la seconde pression expiratoire positive qui varie dans le temps pour produire un volume de poumon ventilé diminué en comparaison avec la seconde étape de ventilation et ledit réglage résultant en la concentration souhaitée de gaz cible.

**3.** Appareil selon l'une des revendications 1 ou 2, cependant que, pendant un processus de lavage en anesthésie, le gaz cible fourni dans la première étape de ventilation est une substance anesthésique qui correspond à un état d'anesthésie superficielle ou non générale et le gaz cible fourni dans la seconde étape de ventilation est une substance anesthésique correspondant à un état d'anesthésie générale plus profond.

**4.** Appareil selon l'une des revendications 1 à 3, cependant que, pendant un processus de rinçage en anesthésie, le gaz cible fourni dans la première étape de ventilation est une substance anesthésique qui correspond à un état d'anesthésie générale plus profond et le gaz cible fourni dans la seconde étape de ventilation est une substance anesthésie correspondant à un état d'anesthésie superficielle ou non générale.

**5.** Appareil selon l'une des revendications 1 à 4 comprenant de plus un commutateur pour commuter entre un système de ventilation fermé et un système de ventilation ouvert pour ventiler un poumon dans la première étape de ventilation dans un système de ventilation fermé ou semi-fermé dans lequel une fraction de gaz inspiré à nouveau est ajoutée dans le système respiratoire et dans la seconde étape de ventilation dans un système de ventilation ouvert.

**6.** Appareil selon la revendication 5, le poumon étant ventilé dans la troisième étape de ventilation dans

**EP 1 753 494 B1**

un système de ventilation fermé ou semi-fermé.

Fig. 1

Fig. 2

A)

| Anesthesia-induced lung collapse (FRC decrement) |

→ Inefficiency of gas exchange due to V/Q inhomogeneity

+

| Re-breathing circuit (expired gas re-inhalation) |

→ Inefficiency of breathing circuit due to dilution effect

Increased TAU

B)

| Alveolar recruitment strategy (FRC increment) |

→ Increase of active area for gas exchange with an improved V/Q

+

| Systematic adjustment of inspiratory gas composition |

→ Improvement of partial pressure gradient for diffusion

+

| Changing re-breathing into a non-re-breathing circuit during ARS (without gas re-inhalation) |

→ Decreased dilution of inspired gas composition

→ Decreased TAU

# Fig. 3

Fig. 4

Fig. 5

Fig. 6

Fig. 7

TLC

NORMAL
(awake)

COLLAPSED
(anesthesia)

DURING ARS
(anesthesia)

AFTER ARS
(anesthesia)

Fig. 8

| Control action / Ventilation stage | Set of ventilation parameters (e.g. PEEP, PIP) (S1) | Supply of **target gas** to the inspiratory gas composition (S2) | Supply of **re-breathed gas** to the inspiratory gas composition (S3) |
|---|---|---|---|
| First ventilation stage | $PEEP_1$, $PIP_1$ → ventilated lung volume $V_1$ | Set fraction of target gas | Set fraction of re-breathed gas |
| | | → actual target gas concentration | |
| Second ventilation stage | ARS $PEEP_2$, $PIP_2$ → ventilated lung volume $V_2 > V_1$ | Change fraction of target gas | Change fraction of re-breathed gas |
| | | → change of actual target gas concentration towards desired target gas concentraion | |
| Third ventilation stage | $PEEP_3$, $PIP_3$ → ventilated lung volume $V_3 < V_2$ | Set new fraction of target gas | Set new fraction of re-breathed gas |
| | | → desired target gas concentration | |

Fig. 9

A) total lung volume

1st ventilation stage — 2nd ventilation stage

time

B) target gas concentration in the inspiratory gas composition

time

C) target gas concentration of blood compartment

actual target gas concentration of blood compartment

time

Fig. 10

total lung
volume

A)

1st ventilation
stage

2nd ventilation
stage

3rd ventilation
stage

time

target gas concentration
in the inspiratory gas
composition

B)

time

target gas concen-
tration of blood
compartment

C)

actual target
gas concentration
of blood
compartment

desired target
gas concentration
of blood compartment

time

# Fig. 11

A) total lung volume

1st ventilation stage | 2nd ventilation stage | 3rd ventilation stage

time

B) target gas concentration in the inspiratory gas composition

time

C) target gas concentration of blood compartment

actual target gas concentration of blood compartment

without ARS

desired target gas concentration of blood compartment

time

Fig. 12

23

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0745405 A1

**Non-patent literature cited in the description**

- **TUSMAN.** Alveolar Recruitment Strategy improves arterial oxygenation during general anaesthesia. *British Journal of Anaesthesia,* 1999, vol. 82 (1), 8-13
- **TUSMAN.** Alveolar recruitment strategy increases arterial oxygenation during one-lung ventilation. *Annals of Thoracic Surgery,* 2002, vol. 73, 1204-1209
- **TUSMAN.** Effects of recruitment maneuver on atelectasis in anesthetized children. *Anesthesiology,* 2003, vol. 98, 14-22
- **TUSMAN.** Lung recruitment improves the efficiency of ventilation and gas exchange during one-lung ventilation anesthesia. *Anesthesia Analgesia,* 2004, vol. 98, 1604-1609
- **TUSMAN.** Deadspace analysis before and after lung recruitment. *Canadian Journal of Anesthesia,* 2004, vol. 51, 718-722